# EUROPEAN PATENT APPLICATION

(11) **EP 3 587 428 A1**
(43) Date of publication of application: **01.01.2020**
(21) Application number: 18757688.9
(22) Date of filing: 21.02.2018
(51) Int. Cl.: C07F 9/53, C07C 49/92, C07F 19/00, C09K 11/06, C07F 5/00

(54) **PHOSPHINE OXIDE COMPOUND, RARE EARTH COMPLEX, AND LIGHT-EMITTING MATERIAL**

(30) Priority: 27.02.2017 JP 2017035041; 31.08.2017 JP 2017167844
(71) Applicant: National University Corporation Hokkaido University, Sapporo-shi, Hokkaido 060-0808 (JP)
(72) Inventor: HASEGAWA Yasuchika, Sapporo-shi Hokkaido 060-0808 (JP); FERREIRA DA ROSA Pedro paulo, Sapporo-shi Hokkaido 060-0808 (JP); NAKANISHI Takayuki, Sapporo-shi Hokkaido 060-0808 (JP); KITAGAWA Yuichi, Sapporo-shi Hokkaido 060-0808 (JP); FUSHIMI Koji, Sapporo-shi Hokkaido 060-0808 (JP)
(74) Representative: Luten, Martin Haaije
(86) International application number: PCT/JP2018/006179
(87) International publication number: WO 2018/155484

(57) **Abstract**

A phosphine oxide compound represented by the following formula (I) and a rare earth complex containing the same are disclosed: 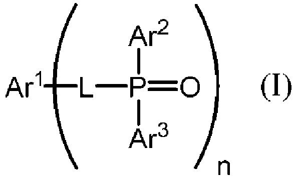 wherein n represents an integer of 3 or more, Ar¹ represents an n-valent aromatic group, L represents a divalent linker group, and Ar² and Ar³ each independently represent a monovalent aromatic groups.

## Description

### Technical Field

The present invention relates to a phosphine oxide compound, a rare earth complex, and a light-emitting material.

### Background Art

Conventionally, various rare earth complexes such as a terbium complex exhibiting emission of green light have been proposed (e.g., Patent Literature 1 and 2).

### Citation List

### Patent Literature

Patent Literature 1: Japanese Unexamined Patent Publication No. 2006-249075
Patent Literature 2: Japanese Unexamined Patent Publication No. 2010-077058

### Summary of Invention

### Technical Problem

A main object of the present invention is to provide a rare earth complex which is capable of emitting light at high luminance and which can be formed into a transparent solid by mixing with a transparent material such as plastic. Use of the rare earth complex which can be formed into a transparent solid enables to easily form a transparent light-emitting material without need of dispersing a complex into a polymer or the like.

### Solution to Problem

In an aspect of the present invention, there is provided a phosphine oxide compound represented by the following formula (I): 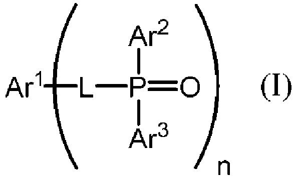 wherein n represents an integer of 3 or more, Ar¹ represents an n-valent aromatic group, L represents a divalent linker group, and Ar² and Ar³ each independently represent a monovalent aromatic group.

Use of the phosphine oxide compound as the ligand of a rare earth complex enables to obtain a rare earth complex which is capable of emitting light at high luminance and which can be formed into a transparent solid by mixing with a transparent material such as plastic.

In another aspect of the present invention, there is provided a rare earth complex comprising the phosphine oxide compound and a rare earth ion coordinated with the phosphine oxide compound. The rare earth complex is capable of emitting light at high luminance and can be formed into a transparent solid. For example, in the case where the rare earth ion is a terbium (III) ion, a rare earth complex which emits green light at high luminance can be obtained.

### Advantageous Effects of Invention

According to the one aspect of the present invention, there is provided a rare earth complex which is capable of emitting light at high luminance and which can be formed into a transparent solid by mixing with a transparent material such as plastic. Further, the rare earth complex in an aspect of the present invention is also capable of having high solubility in a solvent and a polymer material.

### Brief Description of Drawings

Figure 1 is an excitation spectra of terbium complexes.
Figure 2 is an emission spectra of terbium complexes.
Figure 3 is a graph showing decay profiles of emission life of terbium complexes.
Figure 4 is a graph showing relations between emission life and temperature of terbium complexes.
Figure 5 is a graph showing Arrhenius plots obtained from the relations between emission life and temperature of terbium complexes.

### Description of Embodiments

Several embodiments of the present invention are described in detail in the following. The present invention, however, is not limited to the following embodiments.

A rare earth complex in an embodiment comprises a phosphine oxide compound represented by the following formula (I) and a rare earth ion:

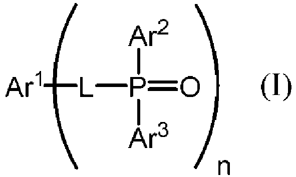

The ratio of a rare earth ion constituting the rare earth complex is typically n equivalent (n mol) to 1 equivalent (1 mol) of the phosphine oxide ligand of formula (I), though not limited thereto. For example, the ratio of the rare earth ion to 1 equivalent of the phosphine oxide ligand of formula (I) may be n×0.5 to n×1.2. With a small ratio of the rare earth ion, a high molecular weight complex described below tends to be formed.

The rare earth ion may be, for example, an ion of rare earth element which is one or two or more selected from the group consisting of Sc, Y, La, Ce, Pr, Nd, Pm, Sm, Eu, Gd, Tb, Dy, Tb, Dy, Ho, Er, Tm, Yb and Lu. The rare earth ion is, for example, a divalent or trivalent cation. The rare earth ion may be one or a combination of two or more selected from the group consisting of a terbium (III) ion (Tb³⁺), a europium (III) ion (Eu³⁺), gadolinium (Gd³⁺), a thulium (III) ion (Tm³⁺), and an erbium (III) ion (Er³⁺). For example, in the case where the rare earth ion is a terbium (III) ion, the rare earth complex generally exhibits emission of green light, and in the case where the rare earth ion is a europium (III) ion, the rare earth complex generally exhibits emission of red light.

In formula (I), n represents an integer of 3 or more, Ar¹ represents an n-valent aromatic group, L represents a divalent linker group, and Ar² and Ar³ each independently represent a monovalent aromatic group. n may be 3 or 4, or may be 3.

The n-valent aromatic group as Ar¹ in formula (I) may be an aromatic hydrocarbon group or a heteroaromatic group. The aromatic group may be a condensed polycyclic group or a group having two or more linked aromatic rings. The n-valent aromatic group may be a group formed by removing n hydrogen atoms from an aromatic compound.

The aromatic hydrocarbon group has, for example, 6 to 14 carbon atoms. Specific examples of the aromatic hydrocarbon group include a group formed by removing n hydrogen atoms from a substituted or unsubstituted benzene, a substituted or unsubstituted naphthalene, a substituted or unsubstituted anthracene, or a substituted or unsubstituted phenanthrene.

Specific examples of the heteroaromatic compound from which the heteroaromatic group is derived include a monocyclic heteroaromatic compound such as azole, oxol, thiophene, pyridine, a pyrylium ion, a thiopyrylium ion, azepine, oxepin, thiepin, imidazole, pyrazole, oxazole, thiazole, imidazoline, pyrazine and thiazine; and a polycyclic heteroaromatic compound such as indole, isoindole, benzimidazole, quinoline, isoquinoline, quinazoline, phthalazine, pteridine, coumarin, chromone, 1,4-benzodiazepine, benzofuran, acridine, phenoxazine and phenothiazine.

The linker group L in formula (I) may be, for example, -C=C-, -CH=CH- or a divalent aromatic group. Specific examples of the aromatic compound from which the aromatic group as L is derived include the same ones as the specific examples of the aromatic compound from which the aromatic group as Ar¹ is derived.

Ar¹ may be, for example, a group represented by the following formula (11) or (12). In the formulas, * represents a bond at which L is bonded.

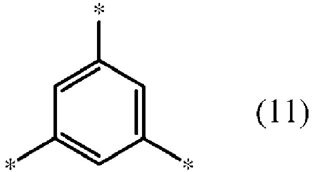

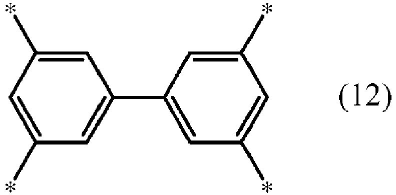

Specific examples of the aromatic compound from which a monovalent aromatic group as Ar² or Ar³ in formula (I) is derived include the same one as the specific examples of the aromatic compound from which an n-valent aromatic group as Ar¹ is derived. In particular, Ar² and Ar³ may be a substituted or unsubstituted phenyl group.

The rare earth complex in an embodiment may further comprise a diketone ligand represented by the following formula (II):

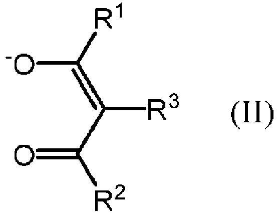

In that case, the rare earth complex may comprise one phosphine oxide coordinated with n rare earth ions (Ln), the n rare earth ions each being coordinated with three diketone ligands, as represented, for example, by the following formula (III):

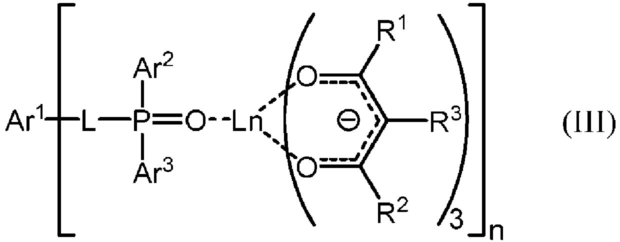

Further, having one rare earth ion coordinated with two or more phosphine oxide ligands of formula (I), for example, a high-molecular weight complex comprising two or more phosphine oxide ligands of formula (I) which are linked through a rare earth ion as shown in the following formula (IV) can be formed. In formula (IV), m (coordination number of diketone ligands) is typically 2, though not limited thereto.

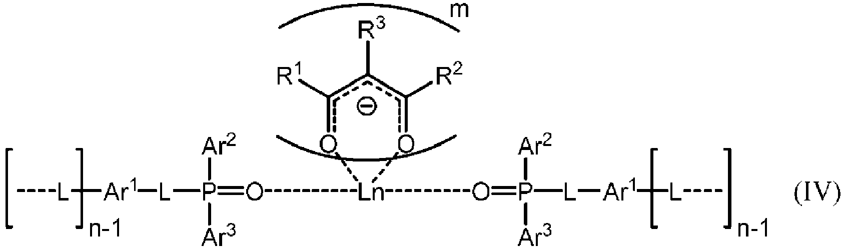

In Formula (II), R¹, R² and R³ each independently represent a hydrogen atom, an alkyl group, a halogenated alkyl group, an aryl group or a heteroaryl group. The alkyl group and the halogenated alkyl group as R¹, R² or R³ may have 1 to 15, 1 to 5, or 1 to 3 carbon atoms. The halogenated alkyl group may be a fluorinated alkyl group. Examples of the aryl group and the heteroaryl group as R¹, R² or R³ include a naphthyl group and a thienyl group.

In particular, R¹ and R² in formula (II) may be a tert-butyl group. A combination of a diketone ligand having tert-butyl groups as R¹ and R² and a terbium (III) suppresses reverse energy transfer during excitation of the complex, so that emission at specifically higher luminance tends to be obtained.

Specific examples of the diketone compound to form the diketone ligand of formula (II) include 2,2,6,6-tetramethylheptane-3,5-dione (tmh), acetylacetone (acac), 1,1,1-trifluoroacetone (TFA), and 1,1,5,5,5-hexafluoro acetylacetone (HFA). Among these, especially 2,2,6,6-tetramethylheptane-3,5-dione (tmh) may be selected from the viewpoints of emission luminance and the like.

The phosphine oxide compound of formula (I) is able to be synthesized, for example, by combining typical reactions known to those skilled in the art with use of a halogenated aromatic compound as starting material.

The rare earth complex of the present embodiment may be synthesized, for example, by a method comprising a step of forming an intermediate complex having a diketone ligand and a rare earth ion through reaction between a rare earth compound as a raw material and a diketone compound from which a diketone ligand of formula (II) is derived, and a step of forming an object rare earth complex through reaction of the intermediate complex and a phosphine oxide compound of formula (I). These reactions may be performed by a method of stirring in a suitable solvent in the presence of a catalyst on an as needed basis. As the solvent, for example, methanol and a mixed solvent of dichloromethane/methanol may be used.

The light-emitting material in an embodiment contains the rare earth complex described above. For example, formation of a light-emitting layer using the light-emitting material is applicable to a light-emitting element such as an LED element and an organic EL element having a light-emitting layer. The light-emitting element is usable in display or lighting. The light-emitting material may be used as light-emitting ink composition. The content of the rare earth complex in a light-emitting material is not particularly limited, and may be, for example, 5 to 100 mass%. A terbium complex is able to form a transparent solid, so that a transparent light-emitting layer is able to be formed therefrom alone.

The light-emitting material may contain a rare earth complex in the present embodiment and a plastic material. The rare earth complex in the present embodiment has excellent solubility, being usable easily as fluorescent substance compounded in various plastic materials.

A light-emitting layer containing a rare earth complex may be formed, for example, by a method comprising a step of depositing a light-emitting material containing a rare earth material and other materials added as needed such as a solvent or a plastic material, to form a light-emitting layer. In the case where the light-emitting material contains a solvent, the solvent may be removed from the light-emitting layer after deposition. The light-emitting layer thus formed may be subjected to heat treatment. Through a heat treatment, the rare earth complex is able to be oriented. Also, through a heat treatment, emission at higher luminance is able to be exhibited. The heat treatment temperature may be, for example, 80 to 150°C. The heat treatment time may be, for example, 1 second to 60 minutes.

### Examples

The present invention will be described in further detail based on Examples as follows. However, the present invention is not limited to the following Examples.

### 1. Synthesis Example

### 1-1. Ligand

### <Monodentate ligand and a bidentate ligand>

Using ethynyl benzene or 1,3-diethylbenzene as raw material, a monodentate ligand diphenylphosphorylethynyl benzene (dpeeb) and a bidentate ligand 1,3-bis[diphenylphosphoryl)ethynyl]benzene (bdppeb) were synthesized by a conventional method.

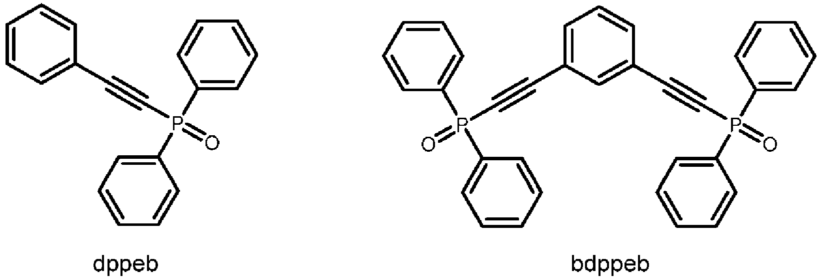

### <Tridentate ligand>

### Synthesis of 1,3,5-triethynyl benzene:

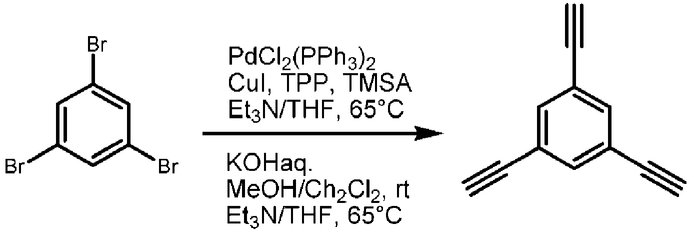

In a flame-dried reaction vessel, a dry tetrahydrofuran solution of triethylene amine was placed under an argon atmosphere. While heating the solution to 65°C, 1,3,5-tribromobenzene (3.21 g, 10 mmol), bis(triphenylphosphine)palladium(II) dichloride (0.36 g, 0.5 mmol), triphenylphosphine (0.28 g, 1.0 mmol), and copper iodide(I) (0.10 g, 0.525 mmol) were added and the solution was stirred for 30 minutes. After the temperature was lowered to 50°C, trimethylsilylacetylene (7.04 mL, 50 mmol) was slowly added and the solution was stirred for 16 hours. Then, 60 mL of brine was added to the reaction vessel and extraction from the reaction liquid was performed three times with dichloromethane. The collected dichloromethane layer was dehydrated with anhydrous MgSO₄, and the solvent was then removed to obtain an oily crude product. The resulting crude product was purified by column chromatography (silica gel, hexane:diethyl ether=5:1). The solvent was removed from the solution containing the product, so that a deep yellow solid was obtained. The solid was dissolved in dichloromethane (30 mL). A mixture of methanol (30 mL)/1M KOH (50 mL, 50 mmol) was slowly added thereto, and the solution was stirred for 12 hours at room temperature. The progress of the reaction was confirmed by TLC. After completion of the reaction, 90 mL of brine was added to the mixture, and extraction from the solution was performed three times with dichloromethane. The collected dichloromethane layer was dehydrated with anhydrous MgSO₄, and the solvent was then removed to obtain 1,3,5-triethynyl benzene (deep brown solid).

### Synthesis of 1,3,5-tris[(diphenylphosphoryl)ethynyl]benzene (tdppeb):

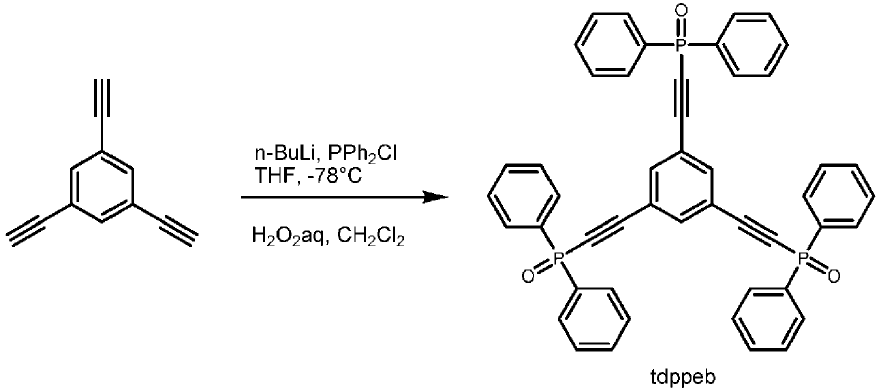

In a flame-dried reaction vessel, dry tetrahydrofuran and 1,3,5-triethynyl benzene (teb, 1.28 g, 8.5 mmol) were placed under an argon atmosphere. While cooling at -80°C, n-butyllithium (21.3 mL, 1.6 M hexane, 34 mmol) was slowly added thereto. After stirring for 3 hours, the reaction liquid was cooled to -80°C again, and chlorodiphenylphosphine (6.3 mL, 34 mmol) was then added to the reaction liquid. The reaction liquid was returned to room temperature and then stirred for 15 hours. After completion of the reaction, 90 mL of brine was added to the reaction liquid, and extraction from the reaction liquid was performed three times with dichloromethane. The solvent was removed from the collected dichloromethane layer. The residual oily product was dissolved in dichloromethane (40 mL). The solution was cooled to 0°C, and 20 mL of an aqueous solution of H₂O₂ at a concentration of 30% was added thereto. The reaction solution was stirred for 3 hours. After completion of the reaction, 90 mL of brine was added to the reaction liquid, and extraction from the reaction liquid was performed three times with dichloromethane. The collected dichloromethane layer was dehydrated with anhydrous MgSO₄, and the solvent was removed to obtain an opaque brown solid. The solid was purified by column chromatography (silica gel, ethyl acetate: methanol=1:20). The product was washed with ethyl acetate to obtain a white powder. The powder was then recrystallized with methanol to obtain tdppeb (transparent crystal).
¹HNMR (400 MHz, CDCl₃, 25°C): δ 7.82-7.89 (m, 15H, -CH), δ 7.56-7.61 (m, 6H, -CH), δ 7.49-7.54 (m,12H, -CH) ppm.
ESI-MS (m/z): calc. for C₄₈H₃₄P₃O₃^{[M+H]+}: 751.17, found 751.17. Elemental analysis calcd (%) for C₄₈H₃₃P₃O₃: C, 76.80; H, 4.43, Found C, 74.69; H, 4.66

### 1-2. Terbium complex

Using the prepared phosphine oxide ligand, the following mononuclear complex and binuclear complex were synthesized. However, the binuclear complex, in particular, may have a structure not limited to that represented by the following formula. There exists a possibility that two or more phosphine oxide ligands may be coordinated with one terbium (III) ion, in such a way as to form a high molecular weight complex with two or more phosphine oxide ligands being linked.

### Mononuclear complex [Tb(tmh)₃(dppeb)]:

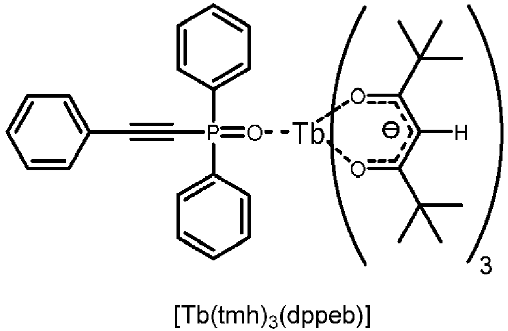

In 60 mL of methanol, 0.29 g (0.4 mmol) of [tris(2,2,6,6-tetramethyl-3,5-heptane-3,4-dionate)]terbium (Tb(tmh)₃(H₂O)) and 0.12 g (0.4 mmol) of dppeb were dissolved. The resulting solution was heated to reflux for 12 hours. Subsequently, after the solution was concentrated with an evaporator, methanol was added thereto and filtered. The filtrate was allowed to stand at room temperature, so that [Tb₃(tmh)₃(dppeb)] was deposited as a transparent crystal.

### Binuclear complex ([Tb₂(tmh)₆(bdppeb)]):

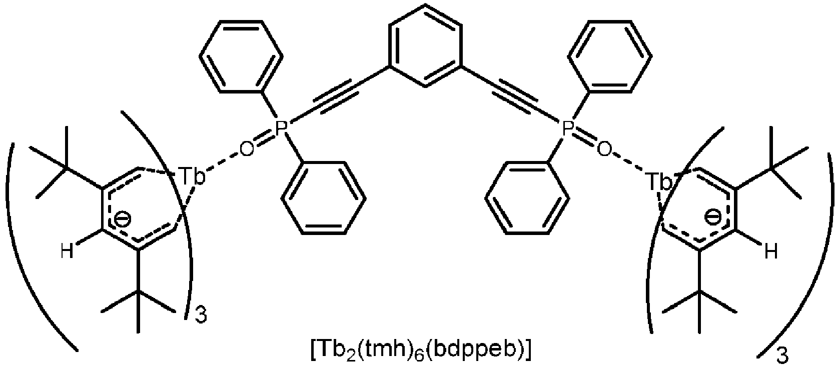

In 60 mL of methanol, 0.58 g (0.8 mmol) of [tris(2,2,6,6-tetramethyl-3,5-heptane-3,4-dionate)terbium (Tb(tmh)₃(H₂O)) and 0.21 g (0.4 mmol) of bdppeb were dissolved. The resulting solution was heated to reflux for 12 hours. Subsequently, after the solution was concentrated with an evaporator, methanol was added thereto and filtered. The filtrate was allowed to stand at room temperature, so that [Tb₃(tmh)₆(dppeb)] was deposited as a pale red transparent spherical crystal.

### 2. Excitation spectrum and emission spectrum

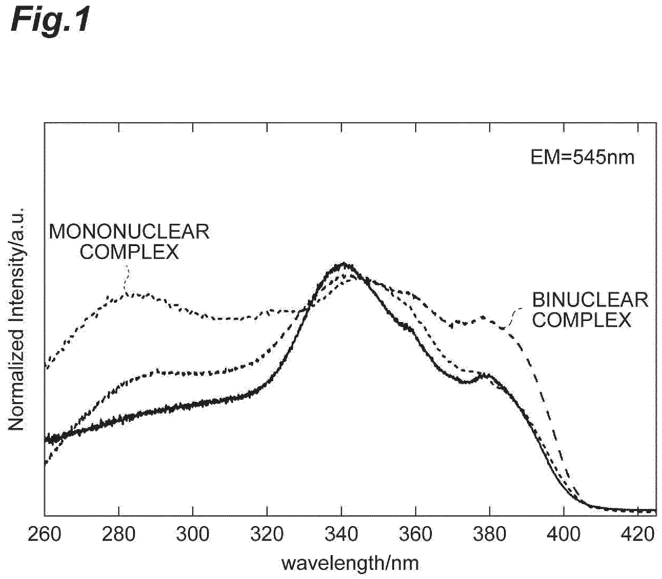

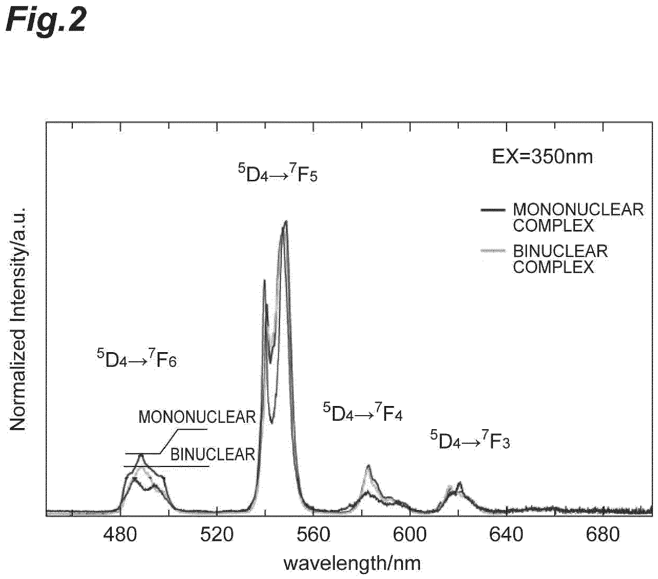

Using a fluorometer, excitation spectra and emission spectra of the powder of the synthesized mononuclear complex and binuclear complex were measured. Figure 1 is a graph showing excitation spectra, and Figure 2 is a graph showing emission spectra. The excitation spectra are normalized at 350 nm, and the emission spectra are normalized at 545 nm. Although all of the three complexes have tmh as a common photosensitizing ligand, different excitation spectra are shown. It is, therefore, suggested that the bridging ligand affects the symmetry around the terbium (III) ion. The emission spectra were spectra corresponding to emission of green light specific to a terbium complex.

### 3. Emission lifetime and luminance

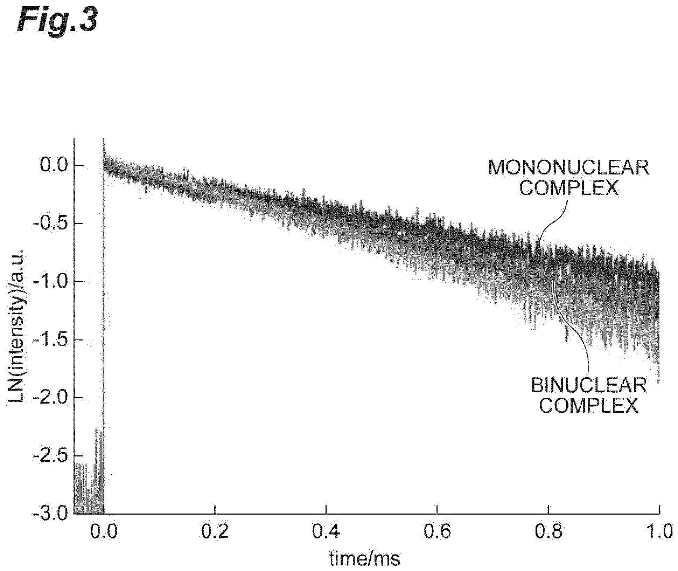

Figure 3 is a graph showing decay profiles of the emission life of a mononuclear complex and a binuclear complex in chloroform at 25°C (excitation light: 355 nm). Table 1 shows the emission life determined from the decay profile, and the emission efficiency measured in chloroform at 25°C (excitation light: 360 nm). In Table 1, an emission life and an emission efficiency of Tb(tmh)₃(TPPO)₂ as mononuclear complex for comparison having triphenylphosphine-oxide (TPPO) as ligand, represented by the following formula, are also shown together.

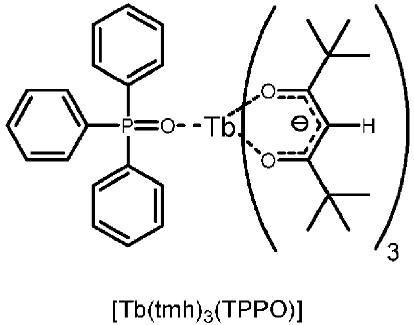

**Table 1**

| | Emission life T_{obs} / ms | Emission efficiency φₜₒₜ / % |
|---|---|---|
| [Tb(tmh)₃(TPPO)] | 0.73 | 66 |
| Mononuclear complex [Tb(tmh)₃(dppeb)] | 1.2 | 71 |
| Binuclear complex [Tb₂(tmh)₆(bdppeb)] | 0.81 | 39 |

### 4. Temperature dependence of emission life

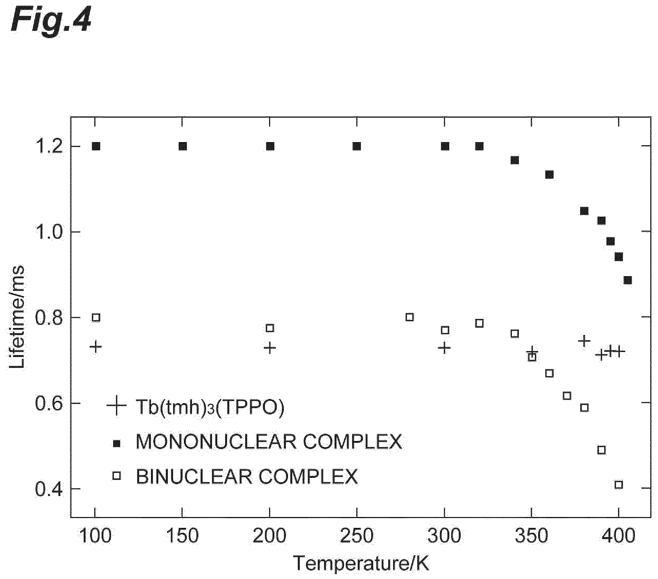

The emission life of a mononuclear complex and a binuclear complex was measured while changing the temperature in the range of 100 to 400 K. Figure 4 is a graph showing relations between emission life and temperature of the mononuclear complex and the binuclear complex. In Figure 4, the emission life of Tb(tmh)₃(TPPO)₂ as a mononuclear complex for comparison is shown as well.

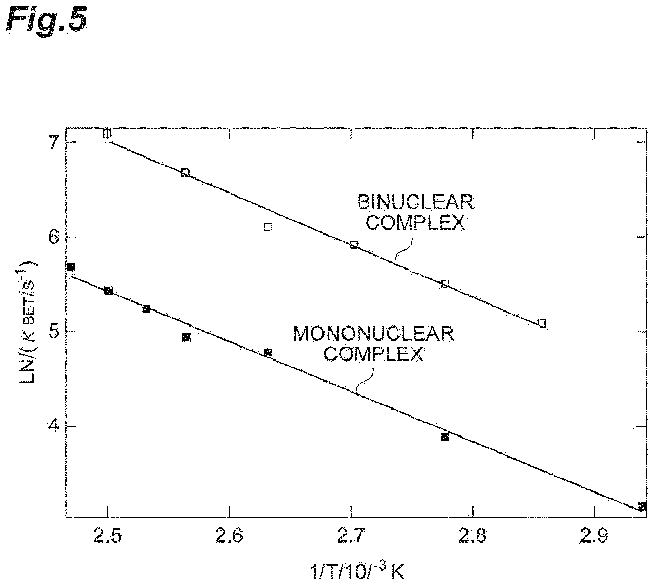

Since Tb(tmh)₃(TPPO)₂ exhibited a nearly constant emission life in the range of 100 to 400 K, it is suggested that the reverse energy transfer from Tb (III) in an excited state to tmh and TPPO in a triplet excited state (T₁) has hardly occurred. On the other hand, Tb(tmh)₃(dppeb) as a mononuclear complex having dpeeb and Tb₂(tmh)₆(bdppeb) as a binuclear complex having bdppeb exhibited a tendency that the emission life has been shortened at a temperature of 320 K or more. In other words, Tb(tmh)₃(dppeb) and Tb₂(tmh)₆(bdppeb) exhibited temperature-sensitive luminescence. Figure 5 is a graph showing Arrhenius plots of a mononuclear complex and a binuclear complex obtained from the measurement results in Figure 4. In Table 2, a frequency factor A obtained from the plot in Figure 5 and an activation energy E for reverse energy transfer are shown. From these results, it is suggested that introduction of a linker group such as ethynyl group facilitates reverse energy transfer. Since efficient reverse energy transfer occurs, it can be said that these complexes are suitable, for example, as a probe of temperature sensors.

**Table 2**

| | Frequency factor A / s⁻¹ | E_{α(BET)} / kJ mol⁻¹ | E_{α(BET)}/cm⁻¹ |
|---|---|---|---|
| Mononuclear complex | 1.12 × 10⁸ | 43.6 | 3645 |
| Binuclear complex | 9.26 × 10⁸ | 45.4 | 3795 |

## Claims

1. A phosphine oxide compound represented by the following formula (I): 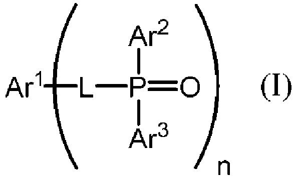 wherein n represents an integer of 3 or more, Ar¹ represents an n-valent aromatic group, L represents a divalent linker group, and Ar² and Ar³ each independently represent a monovalent aromatic group.

2. The phosphine oxide compound according to claim 1, wherein L is -C≡C-, -CH=CH- or a divalent aromatic group.

3. A rare earth complex comprising:
the phosphine oxide compound according to claim 1 or 2; and
a rare earth ion coordinated with the phosphine oxide compound.

4. The rare earth complex according to claim 3, further comprising a diketone ligand coordinated with the rare earth ion and represented by the following formula (II): 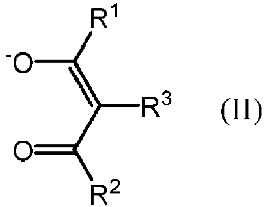 wherein R¹, R² and R³ each independently represent a hydrogen atom, an alkyl group, a halogenated alkyl group, an aryl group or a heteroaryl group.

5. The rare earth complex according to claim 4, wherein R¹ and R² are tert-butyl groups.

6. The rare earth complex according to any one of claims 3 to 5, wherein the rare earth ion is a terbium (III) ion.

7. A light-emitting material comprising the rare earth complex according to any one of claims 3 to 6.

8. A method for producing a light-emitting layer, comprising:
a step of depositing the light-emitting material according to claim 7 to form a light-emitting layer; and
a step of heat-treating the light-emitting layer to orient the rare earth complex.
